# EUROPEAN PATENT APPLICATION

(11) **EP 3 248 565 A1**
(43) Date of publication of application: **29.11.2017**
(21) Application number: 16788409.7
(22) Date of filing: 06.07.2016
(51) Int. Cl.: A61C 8/00, A61C 19/06, A61L 27/56

(54) **POCKET-TYPE MEMBRANE FOR IMPLANT PROCEDURE**

(30) Priority: 10.03.2016 KR 20160028991
(71) Applicant: An, Pil Ho, Dong-gu Daejeon 34582 (KR)
(72) Inventor: An, Pil Ho, Dong-gu Daejeon 34582 (KR)
(74) Representative: Hocking, Adrian Niall
(86) International application number: PCT/KR2016/007291
(87) International publication number: WO 2017/155163

(57) **Abstract**

The present invention relates to a membrane for implant, which is a pocket type. In the present invention, the storage space (50) for storing the artificial bone (4) is formed inside the membrane and porous holes (31) is formed at the rear portion (30) in contact with the alveolar bone, thereby facilitating the integration with and regeneration of the alveolar bone tissue in contact with the grafted artificial bone and preventing the deviation of the artificial bone during the procedure and the regeneration afterwards for effective implant.

## Description

### Technical Field

The present invention relates to a membrane for implant, which is a pocket type, allowing artificial bones to be accommodated therewithin and thereby preventing deviation of the grafted artificial bone as much as possible and thus making the artificial bone seated at the correct site as designed to allow alveolar bone regeneration.

### Background Art

Recently, an artificial tooth implant surgery (hireinafter, implant) is widely used for periodontal disease patients. In this case, a fixed prosthesis is widely used for anodontia patients.

However, during the implant, since it does not ensure sufficient width and length of the maxilla and mandible owing to various anatomical limitations, it cannot conduct the implant.

At this time, if it applies a guided bone regeneration, since the placement of the implant is possible, it can help to ensure a long-term stability.

In this case, a membrane for guided bone regeneration is used. That is, since the membrane for guided bone regeneration serves to selectively repopulate the cells, it is used for the independent reproducing of each organization during the healing period.

That is, after the transplant operation of artificial bones, since it can prevent the disturbance of bony tissue formation owing to the infiltration of cells of other tissues between the artificial bone and the alveolar bone, it serves to stably induce the bony tissue cells.

In the initial research on the membrane, it is mainly interested in using the membrane for the treatment of periodontal defect. Presently, it is used for increasing the augmentation of alveolar ridge, improving the curing of peri-implant bone, inducing the complete bone regeneration, opening the results of bone graft, and treating the failed implant.

If it uses the membrane, it can prevent the unwanted tissue, especially connective tissue from incoming to the place intended for bone regeneration. Accordingly, it serves to prevent the disturbance of the bone formation, additionally stabilize and protect the blood clot by acting as a surgical flap for covering wound, and prevent the destruction of the blood clot along the interface between the healing tissue and the surface of the tooth root.

In addition, the membrane serves to form a space such as a tent, so that the blood clot can be kept in the lower portion thereof. Also, the blood clot can serve as a fat for in-growth of cells and blood vessel derived from the vascular defect base.

Therefore, the membrane for guided bone regeneration serves to create an environment capable of maximizing the natural and biological capacity for the functional regeneration, form and maintain the space filled with the blood clot so as to prevent the infection of bacteria, separate from the unwanted tissue, and mechanically stabilize the wound healing complex.

Referring to "membrane for guided bone regeneration and preparing method thereof" (Korean Patent registration No. 10-0738476, Patent Literature 1) as the technique related to such a membrane, a rectangular membrane is trimmed in a position, that a bone defect is generated, in a proper shape.

However, in this technique, after it recognizes the bone defect shape, since the membrane should be trimmed in the required shape, there are problems in that it is difficult to perfectly cover the bone defect portion and the treatment time is increased.

Also, where a screw for fixing the membrane is used, since it conduct a wide gingival incision for screw installation, there are problems in that the operating time is increased and it gives a burden to the operator and the patient owing to an edema generated from the bleeding.

In order to solve this problem, "Membrane for regenerating alveolar bone" (Korean Patent registration No. 10-1182806, Patent Literature 2) discloses a membrane for regenerating alveolar bone of forming a center hole, through which an implant insert passes, and taking a rounded shape overall.

In the Patent Document 2, the membrane is fixed in such a manner that the implant insert passes through the hole.

By the way, the bone graft is mostly operated in case of implant insert difficulty. Also, it is conducted before the insertion of the implant insert. Accordingly, the technique of the Patent Literature 2 is not suitable for the treatment method of conducting the implant after the bone graft.

### Patent Literature

Patent Literature 1: Korean Patent Registration No. 10-0738476 (July 05, 2007)
Patent Literature 2: Korean Patent Registration No. 10-1182806 (September 07, 2012)

### Disclosure

### Technical Problem

The present invention has been made in an effort to solve the problems of the conceptual description of the conventional art as described above, and an object of the present invention is to provide a pocket type membrane for implant in that the membrane is formed into a pocket shape in which an artificial bone is stored, to prevent deviation of the artificial bone during and after artificial bone grafting and thereby to allow the outcome of artificial bone grafting to approach the desired design as closely as possible.

Also, in the present invention, the portion in contact with the alveolar bone has a porous structure whereas the other portion of the pocket has a closed structure, so as to facilitate integration of the artificial bone accommodated in the pocket with and regeneration of the alveolar bone while preventing deviation of the artificial bone to the outside of the pocket.

In addition, the pocket type membrane for implant according to the present invention is provided with wing portions protruding toward both sides of the front portion to prevent deviation of artificial bone by means of screw coupling to the adjacent alveolar bone.

Further, the pocket type membrane for implant according to the present invention is provided with a cover portion extending from a front portion to a rear portion to prevent deviation of artificial bone through an opening at the top.

In addition, creases at the side portions of the membrane of the present invention enable adding or subtracting the amount of artificial bones according to the need before storage of the artificial bone.

### Technical solution

According to one aspect of the present invention so as to accomplish these objects, there is provided to a pocket type membrane for implant, including: a front portion (10); side portions (20) connected to both sides of the front portion (10); a rear portion (30) connected to the side portions (20) and formed at the rear of the front portion (10) so as to contact the alveolar bone of the person to be operated on, on the surface of which a plurality of holes (31) passing therethrough are formed; and a bottom portion (40) whose circumference is connected to the lower part of the front portion (10), the lower part of the side portions (20) and the lower part of the rear portion (30) to form an upwardly extending storage space (50) enclosed by the front portion (10), the side portions (20) and the rear portion (30).

In the above construction, in the boundaries between the front portion (10) and the side portions (20), wing portions (60) are formed protruding toward both sides with respect to the front portion (10).

Also, a cover portion (70) which covers an upper part of the storage space (50) and an end of which extends to a back of the rear portion is formed at a top of the front portion (10).

Also, creases (21) are formed at the side portions (20) in a vertical direction.

Also, the rear portion (30) is made of a biodegradable material.

Also, the side portion (20) is made of a biodegradable material.

Also, the bottom portion (40) is made of a biodegradable material.

### Advantageous Effects

According to the present invention, the membrane is formed into a pocket shape in which an artificial bone is stored, to prevent deviation of the artificial bone during and after artificial bone grafting and thereby to allow the outcome of artificial bone grafting to approach the desired design as closely as possible.

Also, the portion in contact with the alveolar bone has a porous structure whereas the other portion of the pocket has a closed structure, so as to facilitate integration of the artificial bone accommodated in the pocket with and regeneration of the alveolar bone while preventing deviation of the artificial bone to the outside of the pocket.

In addition, it is provided with wing portions protruding toward both sides of the front portion to prevent deviation of artificial bone by means of screw coupling to the adjacent alveolar bone.

Further, it is provided with a cover portion extending from a front portion to a rear portion to prevent deviation of artificial bone through an opening at the top.

In addition, the creases at the side portions thereof enable adding or subtracting the amount of artificial bones according to the need before storage of the artificial bone.

### Brief Description of Drawings

The above and other objects, features and advantages of the present invention will be more apparent from the following detailed description taken in conjunction with the accompanying drawings, in which:
Figure 1 illustrates one example of the site where the pocket type membrane for implant according to the present invention is installed;
Figure 2 is a sectional view illustrating a procedure of an artificial bone grafting using the pocket type membrane for implant according to the present invention;
Figure 3 is a perspective view illustrating a basic embodiment of the pocket type membrane for implant according to the present invention;
Figure 4 is a perspective view illustrating an example in that creases are formed at the pocket type membrane for implant according to the present invention;
Figure 5 is a perspective view illustrating an example in that a cover portion is formed at the pocket type membrane for implant according to the present invention;
Figure 6 is a perspective view illustrating an example in that wing portions are formed at the pocket type membrane for implant according to the present invention; and
Figure 7 is a perspective view illustrating an example in that auxiliary wing portions are formed at the pocket type membrane for implant according to the present invention.

### [Reference Signs List]

1: teeth
2: gum
2a: incision
3: alveolar bone
3a: groove portion
4: artificial bone
10: front portion
20: side portion
21: creases
30: rear portion
31: hole
40: bottom portion
50: storage space
60: wing portions
61: auxiliary wing portions
70: cover portion

### Best Mode

### Mode for Invention

Hereinafter, a preferred embodiment according to the present invention will be described in detail with reference to the accompanying drawings.

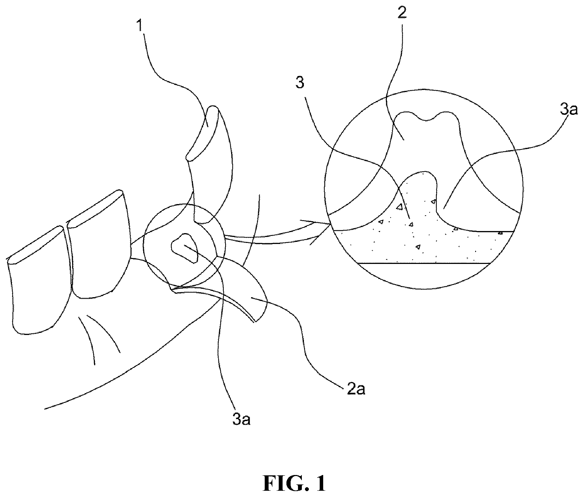

Figure 1 illustrates one example of the site where the pocket type membrane for implant according to the present invention is installed.

From the drawing, it can be understood that the alveolar bone (3) inside an incision (2a) made by cutting the gum (2) at the site of the intended implant between adjacent teeth (1) is composed of a structurally unstable groove portion (3a) like a groove to the extent that it is unsuitable for insertion and stabilization of an inserted structure for implant procedure.

The enlarged cross-sectional diagram illustrates one example of this groove portion (3a).

When the alveolar bone (3) is not wide enough as in this case, artificial bone grafting is generally conducted for several months to regenerate and integrate the alveolar bone until the alveolar bone becomes wide enough as the adjacent alveolar bone, before an implant structure is placed.

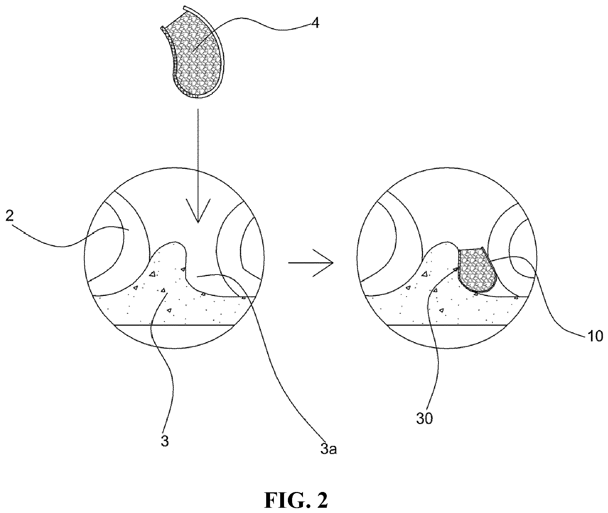

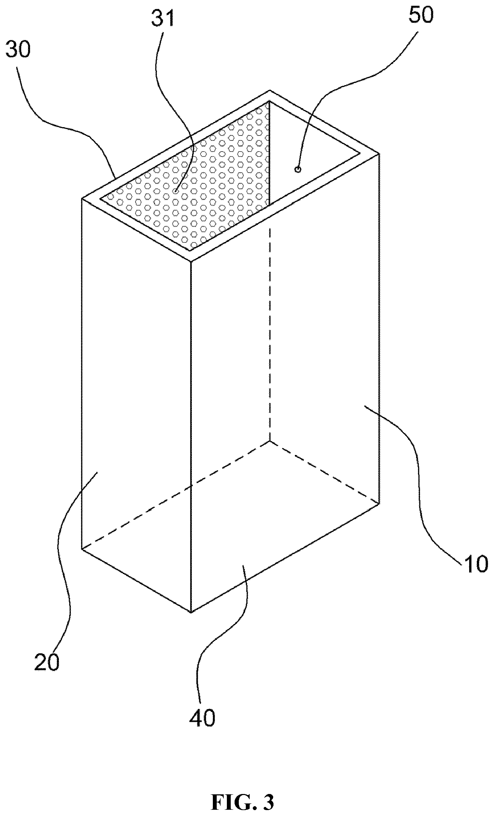

Figure 2 and Figure 3 illustrate a method of procedure using the pocket type membrane for implant according to the present invention and a basic embodiment of the pocket type membrane for implant according to the present invention.

The exemplary shape shown in Figure 3 comprises a front portion (10); side portions (20) connected to both sides of the front portion (10); a rear portion (30) connected to the side portions (20) and formed at the rear of the front portion (10) so as to contact the alveolar bone of the person to be operated on, on the surface of which a plurality of holes (31) passing therethrough are formed; and a bottom portion (40) whose circumference is connected to the lower part of the front portion (10), the lower part of the side portions (20) and the lower part of the rear portion (30) to form an upwardly extending storage space (50) surrounded by the front portion (10), the side portions (20) and the rear portion (30).

The front portion (10), the side portions (20), and the bottom portion (40) have a closed structure which prevents the deviation of the artificial bone stored in the storage space (50) to the outside, and the rear portion (30) has a plurality of holes (31) formed thereon to facilitate the integration with and regeneration of the alveolar bone tissue in contact.

In the procedure according to Figure 2, the gum (2) around the groove portion (3a) having an unstable shape is first cut, and artificial bone is packed into the storage space (50) of the pocket type membrane for implant according to the present invention. After that the membrane is placed and fixed in the groove portion (3a), and then the incised gum (2) is closed with stitches to finish the procedure.

Figure 2 shows that the rear portion (30) with a plurality of holes (31) formed therein is in contact with the portion forming the wall surface of the groove portion (3a), and the adjacent bottom portion (40), side portions (20) and front portion (10) enclose the storage space (50) to make the artificial bone (4) stably placed while preventing the deviation thereof.

Here, the artificial bone (4) and the existing alveolar bone (3) are integrated with each other through the holes (31) to facilitate regeneration.

In particular, storage of the artificial bone (4) in the membrane in a pocket shape allows the outcome of regeneration of the alveolar bone for several months to approach as closely as possible to the intended alveolar bone generation.

The material for the pocket type membrane for implant according to the present invention composed of the above-described components may be **r**esorbable or non-resorbable depending on the method of the procedure performed.

Examples of the non-resorbable material include titanium thin film having micropores and expanded polytetrafluoroethylene.

These materials have been used for the first-generation membrane and are known to be compatible with bone.

Especially, ePTFE, which refers to a film obtained by processing PTFE with a ram extruder and stretching it biaxially to add porosity to the inside thereof, has a low friction coefficient and thus has excellent antithrombotic properties.

However, ePTFE has a drawback that a second surgery is needed to remove the tissues after the tissue regeneration.

The non-resorbable material may also be made of collagen, as collagen is a macromolecule physiologically metabolized in the periodontal connective tissue.

Besides, a two-layer membrane consisting of a slowly decomposing outer layer and an inner layer including heparin sulfate or fibronectin (Ossix manufactured by Colbar R&D Ltd. in Israel) may be used.

Examples of the resorbable membrane include a resorbable membrane obtained by adding citric acid to polylactic acid to increase the flexibility and operability.

Examples of the material of the resorbable membrane also include PLA, PGA and PLGA. However, since they have a high glass transition temperature and thus are brittle at the room temperature or at the in vivo temperature, it is difficult to form a thin, flexible film by using them. Thus, in case of using them as a raw material of the membrane, they may be formed into a fiber and then into a non-woven or woven fabric before use.

The preferred composition of the materials is as follows: a biodegradable material with a degradation period of about 6 months for the bottom portion (40), the side portions (20) and the front portion (10), and a biodegradable material with a degradation period of about 1 month for the rear portion (30), so that the grafted artificial bone's integration with, and regeneration of, the alveolar bone is well facilitated while being protected from the outside during the regeneration period.

The holes (31) are differentiated from the pores presented in Patent Document 1. The pores of Patent Document 1 are 10 to 50*µ*m in size which makes it capable of preventing the circumferential soft tissue from penetrating into the region of bone graft material while the bone is stably growing, and thus inhibiting the movement of epithelial cells toward the root apex.

In contrast, the holes (31) of the present invention are not formed in the front portion (10), which corresponds to the region of contact between epithelial cells and the root apex side, but in the region where the alveolar bone in the root apex side and the artificial bone stored in the storage space (50) of the membrane of the present invention contact with each other. Thus, the holes may be of any size as long as they can prevent deviation of the artificial bone stored in the storage space (50) during the procedure and as long as it allows, after the procedure, the artificial bone to grow and integrate well with the existing alveolar bone. Thus, the holes (31) may have any size as long as it is smaller than the diameter of the artificial bone.

It does not matter whether the material of the membrane of the present invention is **r**esorbable or non-resorbable. However, it is preferred that the rear portion (30) be composed of a **r**esorbable material so that it can be resorbed into the body after the growth of artificial bone is completed.

However, portions other than the rear portion (30) may be composed of a resorbable (biodegradable) material or a non-resorbable material depending on the necessity of membrane removal surgery.

Particularly, as can be understood from the state of the procedure shown in the drawing, depending on the manner of arrangement of the membrane of the present invention, portions other than the rear portion (30) may also partially contact the alveolar bone.

Thus, either or both of the side portions (20) and bottom portion (40) may be partially or wholly composed of a biodegradable material.

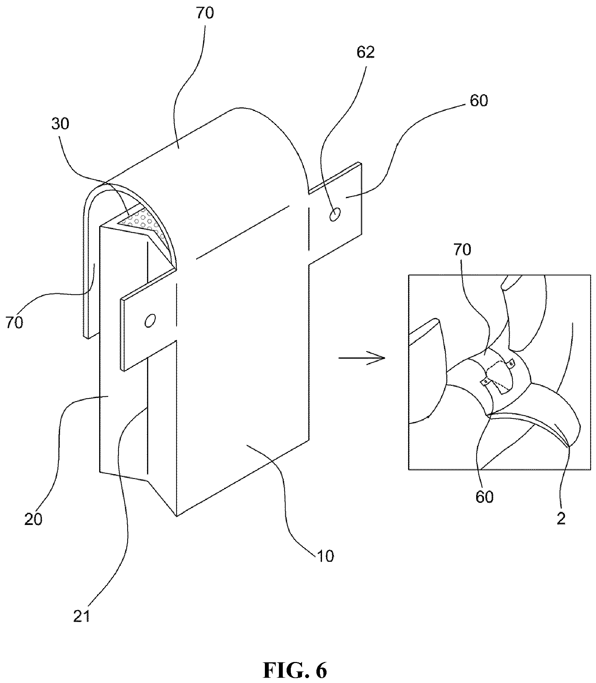

In the above-described constitution, wing portions (60) protruding toward both sides of the front portion may be added as illustrated in Figure 6.

Figure 6 shows that, in the boundaries between the front portion (10) and the side portions (20), wing portions (60) are formed protruding toward both sides with respect to the front portion (10).

As can be understood from the part in Figure 6 showing the state of procedure, these wing portions (60) allow to fix both sides of the membrane of the present invention to the gum or the alveolar bone located at both sides of the site of procedure by using a screw, a clamp, or a bonding material, so that the position of the membrane is stably fixed during the procedure and the regeneration afterwards.

In the case where a procedure is performed with a method of placing a screw into each of the wing portions (60), a procedure for removing the screw is required. However, in the case of using a bonding material, a method that does not require a reoperation may be sought.

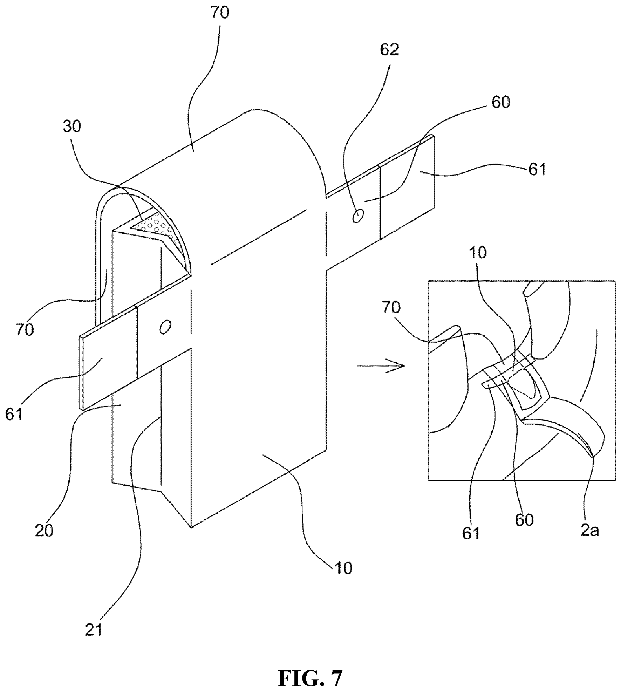

In Figure 6 and Figure 7, reference numeral 62 indicates a screw engagement hole for engagement of screws.

The drawings show an example where the hole is formed in each wing portion (60), but the present invention is not limited thereto. The screw engagement hole may also be formed in a cover portion (70) or auxiliary wing portions (61), which will be described below.

When the fixation is performed with a screw, the wing portions (60) are located within the gum after a suturing procedure, and the wing portions (60) may be composed of a biodegradable material.

Here, the screw may also be composed of a biodegradable material as needed so as not to perform a removal surgery.

On the other hand, in case of seeking a method that does not require a reoperation, the front portion (10) and the wing portions (60) must be composed of a biodegradable, i.e., resorbable material. In this case, after the material is resorbed into the body, it also loses the function as a bonding agent for fixing the wing portions (60) to the gum.

In order to resolve this problem, auxiliary wing portions (61) may be formed connected to the end of each of the wing portions (60), protruding to the outside of the incision (2a) of the gum (2) to be sutured after a procedure, and composed of a nondegradable material, as illustrated in Figure 7.

The auxiliary wing portions (61) may be attached to the gum in contact therewith by bonding with a bonding agent.

When the auxiliary wing portions (61) are provided, the membrane has an advantage of firm fixation immediately after a procedure has been completed, since basically each of the wing portions (60) and the auxiliary wing portions (61) are attached to the gum by bonding.

Also, the auxiliary wing portions (61) allow minimizing the movement of the membrane even during resorption of the biodegradable wing portion (60) into the body, since the auxiliary wing portions (61) are connected to the remaining unresorbed wing portions (60) and fixed to the gum during the resorption into the body.

The auxiliary wing portions (61) and the wing portions (60) may be connected by a perforated line, which resolves the problem that when an end of the auxiliary wing portions (61) is slightly inserted into the healing gum during the healing of the incision (2a) after a suturing procedure is performed, it is difficult to remove the auxiliary wing portion (61).

In the meantime, as shown in Figure 2, after cutting the gum, artificial bone is packed into the storage space (50) of the membrane of the present invention and then the membrane is placed at the site of procedure and sutured. The position of the membrane is fixed during the procedure, and after the procedure, the artificial bone may directly contact the soft tissue above the alveolar bone due to an open space at the upper part of the storage space (50), resulting in penetration of the soft tissue.

In order to resolve this problem, a cover portion (70) which covers the upper part of the storage space (50) and the end of which extends to the back of the rear portion (30) may be formed at the top of the front portion (10).

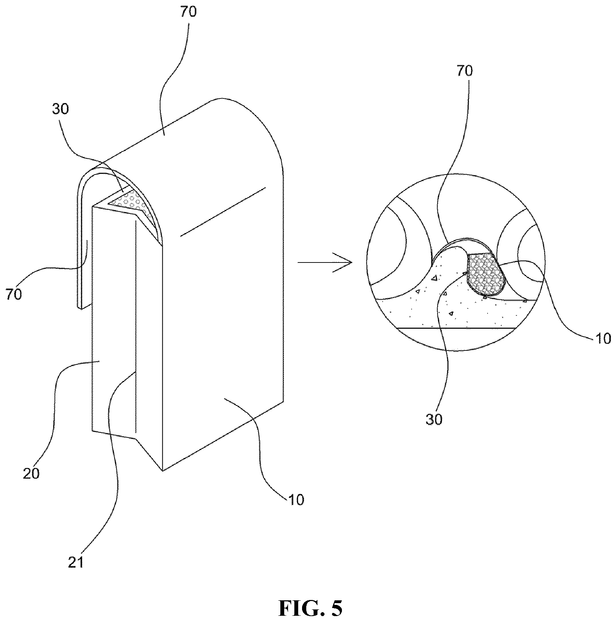

As shown in Figure 5 and Figure 6, application of the membrane using the cover portion (70) allows protection on the upper part of the storage space (50), thereby preventing penetration of the soft tissue into the artificial bone through the upper part of the storage space (50).

In addition, the cover portion (70) enables to prevent penetration of bacteria or foreign substances into the artificial bone stored in the storage space (50) during the procedure, to inhibit penetration of blood resulting from the incision of the gum, and to precisely adjust the position of the membrane during the procedure.

It is preferred that this cover portion (70) be composed of the same material as that of the front portion (10) and formed extending therefrom.

Meanwhile, unlike the conventional membranes, the membrane of the present invention is pocket type and therefore the amount of the artificial bone to be grafted varies depending on the person to be operated on. Thus, in case of customizing the membrane so that the size of the storage space (50) of the membrane fits each person to be operated on, the cost is unduly high.

In the case of a procedure using the membrane with a certain size due to this problem, an empty space may remain in the storage space (50) of the membrane after artificial bone is packed thereinto, and there may be cases where an unnecessarily large region of the gum needs to be incised due to the size of the membrane when the site of procedure is small.

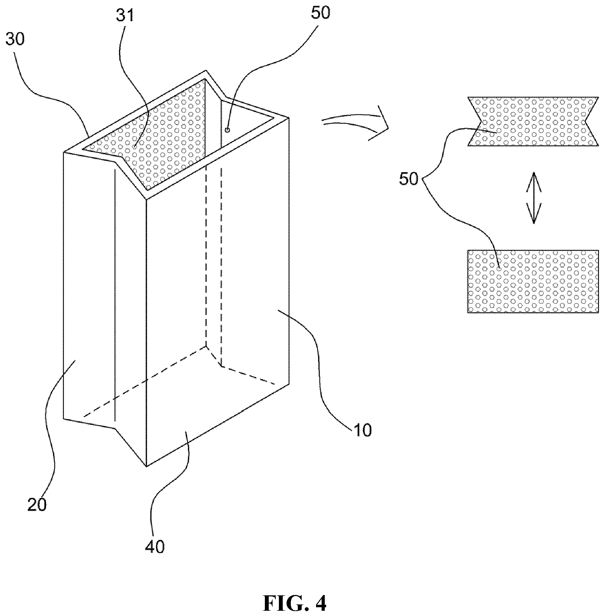

In order to resolve these problems, one or several creases (21) may be formed at the side portions (20) in the vertical direction, as illustrated in Figure 4 to Figure 6.

Besides, although not illustrated in the drawings, a plurality of creases connecting the side portions (20) and the front portion (10) may be formed in the horizontal direction.

In the case where creases (21) are formed as described above, it is possible to fold the membrane by using the creases, which enables to use a membrane of a certain dimension and decrease or increase the volume of the storage space (50) of the membrane as needed according to the amount of the artificial bone stored in the storage space (50), as illustrated in the top plan view of Figure 4.

This makes it possible to incise only the region of the gum to be operated, resulting in an effective procedure.

While the present invention has been described with respect to the specific embodiments, it will be apparent to those skilled in the art that various changes and modifications may be made without departing from the spirit and scope of the present invention as defined in the following claims.

### Industrial Applicability

The pocket type membrane for implant according to the present invention can be used in an artificial bone graft procedure when an artificial bone is grafted into a region where the alveolar bone is unstable to the extent that it is difficult to fix an implant fixture in a dental implant insertion procedure

## Claims

1. A pocket type membrane for implant, comprising:
a front portion (10);
side portions (20) connected to both sides of the front portion (10);
a rear portion (30) connected to the side portions (20) and formed at the rear of the front portion (10) so as to contact the alveolar bone of the person to be operated on, on the surface of which a plurality of holes (31) passing therethrough are formed; and
a bottom portion (40) whose circumference is connected to the lower part of the front portion (10), the lower part of the side portions (20) and the lower part of the rear portion (30) to form an upwardly extending storage space (50) enclosed by the front portion (10), the side portions (20) and the rear portion (30).

2. The pocket type membrane for implant as claimed in claim 1, wherein in the boundaries between the front portion (10) and the side portions (20), wing portions (60) are formed protruding toward both sides with respect to the front portion (10).

3. The pocket type membrane for implant as claimed in claim 1, wherein a cover portion (70) which covers an upper part of the storage space (50) and an end of which extends to a back of the rear portion is formed at a top of the front portion (10).

4. The pocket type membrane for implant as claimed in claim 1, wherein creases (21) are formed at the side portions (20) in a vertical direction.

5. The pocket type membrane for implant as claimed in claim 1, wherein the rear portion (30) is made of a biodegradable material.

6. The pocket type membrane for implant as claimed in claim 5, wherein the side portions (20) are made of a biodegradable material.

7. The pocket type membrane for implant as claimed in claim 6, wherein the bottom portion (40) is made of a biodegradable material.
